(19) Europäisches Patentamt
European Patent Office
Office européen des brevets

(11) EP 2 325 187 A1

(12) **EUROPEAN PATENT APPLICATION**
published in accordance with Art. 153(4) EPC

(43) Date of publication:
**25.05.2011 Bulletin 2011/21**

(21) Application number: **09804444.9**

(22) Date of filing: **31.07.2009**

(51) Int Cl.:
***C07D 495/04*** *(2006.01)* ***A61K 31/4365*** *(2006.01)*
***A61P 7/02*** *(2006.01)*

(86) International application number:
**PCT/CN2009/000860**

(87) International publication number:
**WO 2010/015144 (11.02.2010 Gazette 2010/06)**

(84) Designated Contracting States:
**AT BE BG CH CY CZ DE DK EE ES FI FR GB GR HR HU IE IS IT LI LT LU LV MC MK MT NL NO PL PT RO SE SI SK SM TR**
Designated Extension States:
**AL BA RS**

(30) Priority: **02.08.2008 CN 200810146101**

(71) Applicant: **Lunan Pharmaceutical Group Corporation**
**Shandong Province 276006 (CN)**

(72) Inventor: **ZHAO, Zhiquan**
**Linyi, Shandong 276005 (CN)**

(74) Representative: **Crowhurst, Charlotte Waveney**
**Potter Clarkson LLP**
**Park View House**
**58 The Ropewalk**
**Nottingham**
**NG1 5DD (GB)**

(54) **THE HYDROSULFATE OF PRASUGREL, ITS PHARMACEUTICAL COMBINATION AND USES THEREOF**

(57) The present invention provides the prasugrel bisulfate of formula (II) and pharmaceutical composition and use thereof. Prasugrel bisulfate of the present invention has good stability, oral absorbability, metabolic activity and platelet aggregation inhibition effect, and low toxicity, and is therefore a promising anticoagulant for preventing or treating diseases associated with thrombosis or embolism.

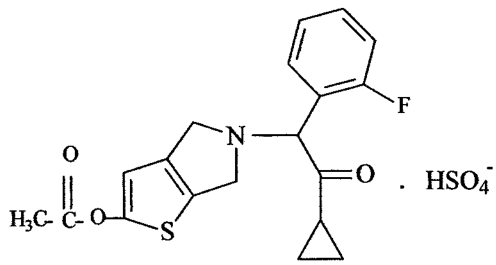

(II)

EP 2 325 187 A1

## Description

### Technical field

The present invention belongs to the field of medicine. Particularly, the present invention is related to a salt of a hydrogenated pyridine derivative, prasugrel bisulfate, and a method for preparing the same, as well as a pharmaceutical composition comprising the same as an active ingredient, and use thereof in preventing or treating diseases associated with thrombosis or embolism.

### Background

Prasugrel is a novel thienopyridine P2Y12 antagonist with the chemical name of 2-acetyloxy-5-(α-cyclopropylcarbonyl-2-fluorobenzyl)-4,5,6,7-tetrahydrothieno[3,2-C ]pyridine and with the following formula (I):

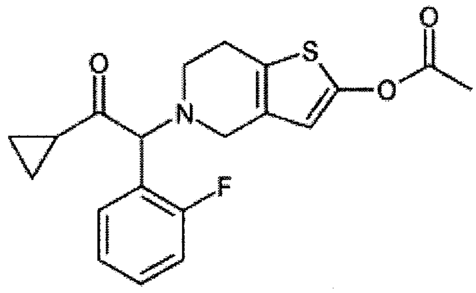

(I)

Developed jointly by Eli Lilly and Company of USA and Sankyo Company of Japan, Prasugrel is a platelet ADP receptor blocker which is still under investigation. Studies demonstrated that prasugrel has a stronger and faster effect on preventing thrombosis than clopidogrel and thus has a better efficacy. After administration, patients in the prasugrel group showed less thrombus in their blood than those in the clopidogrel group, and the rate of developing ischemia in patients in the prasugrel group is lower than that in the clopidogrel group. Therefore, the platelet aggregation inhibition effect of prasugrel is significant and quick. Studies conducted by Johns Hopkins University obviously demonstrated that prasugrel is more promising in inhibiting platelet aggregation than clopidogrel. Prasugrel has a stronger effect in inhibiting platelet aggregation induced by ADP than clopidogrel at currently approved doses. In addition, in a clinical study of prasugrel, which is the second phase of the study with the code JUMBO-TIMI26, prasugrel did demonstrate faster and more uniform platelet inhibition effect than clopidogrel.

Generally, a medicinal compound is used in the form of a pharmaceutically acceptable salt. It is the same for drugs inhibiting platelet aggregation, such as the compound of formula (I). This renders it very important to prepare pharmaceutically acceptable salts of this kind of compounds.

EP1298132 (acid addition salt of hydrogenated pyridine derivatives) discloses 2-acetyloxy-5-(α-cycloproylcarbonyl-2-fluorobenzyl)-4,5,6,7-tetrahydrothieno[3,2-C] pyridine hydrochloride and maleate, method for preparation thereof, and use thereof in inhibiting thrombosis. Although prasugrel sulfate is mentioned in the list of pharmaceutical acid addition salts in the specification of this application, prasugrel bisulfate is disclosed.

EP0542411 discloses a hydrogenated pyridine derivative and a method for preparing its tautomers, in which the hydrogenated pyridine derivative is the parent structure of prasugrel, while prasugrel bisulfate and methods for its preparation are not disclosed.

US2004024013 discloses a pharmaceutical composition comprising 2-acetyloxy-5-(α-cycloproylcarbonyl-2-fluorobenzyl)-4,5,6,7-tetrahydrothieno[3,2-c] pyridine or pharmacologically acceptable salts thereof and aspirin as active ingredients.

In addition, through experiments, the inventors found that prasugrel sulfate, which has the most similar structure to that of prasugrel bisulfate, is extremely unstable, and is very difficult to be prepared. Therefore, it has become a technical problem in the art to provide a pharmaceutical salt of prasugrel which is more stable and has lower side effects.

### Summary

Directing to the above problems of the prior art, the present invention provides prasugrel bisulfate. Prasugrel

bisulfate of the present invention has good stability, oral absorbability, metabolic activity and platelet aggregation inhibition effect, and low toxicity, and is therefore a promising anticoagulant for preventing or treating diseases associated with thrombosis or embolism.

Prasugrel bisulfate of the present invention is a compound represented by the following formula (II):

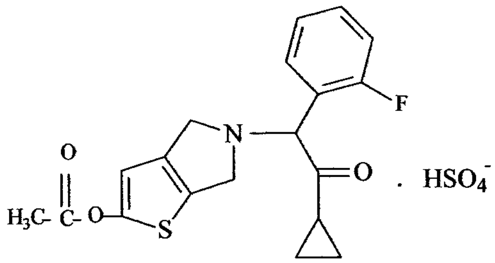

(II)

Prasugrel bisulfate has an asymmetric chiral carbon atom, and therefore has stereoisomers with optical activity. In one embodiment of the present invention, various optical isomers of prasugrel bisulfate may exist individually or in a form of mixture of optical isomers in any proportion. The optical isomers of prasugrel bisulfate may be synthesized with resolved raw materials.

Prasugrel bisulfate may absorb water to form a hydrate while standing in air or during preparation. In one embodiment of the present invention, prasugrel bisulfate may be in a form of a hydrate of prasugrel bisulfate.

In one embodiment of the present invention, the structural parameters of prasugrel bisulfate are demonstrated as follows:

(1)

Table 1-1 Percent content of the elements C, H, N, S, F in prasugrel bisulfate

| Elements | Theoretical Values |
|---|---|
| C | 51.05 |
| H | 4.50 |
| N | 2.98 |
| S | 13.63 |
| F | 4.04 |

(2)

Table 2-2 UV spectral data of prasugrel bisulfate

| Solvents | $\lambda_{max}$/nm | $\varepsilon_{max}$/L·mol$^{-1}$·cm$^{-1}$ | Peak Assignment |
|---|---|---|---|
| Water | 212 | $3.9\times10^3$ | $E_2$ bond of substituted benzene ring |
| | 259 | $1.5\times10^3$ | B bond of substituted benzene ring |
| 0.1 mol/L | 213 | $4.0\times10^3$ | $E_2$ bond of substituted benzene ring |
| HCl | 213 | $1.2\times10^3$ | B bond of substituted benzene ring |
| 0.1 mol/L | 214 | $4.4\times10^3$ | $E_2$ bond of substituted benzene ring |
| NaOH | 259 | $1.3\times10^3$ | B bond of substituted benzene ring |
| Methanol | 214 | $4.8\times10^3$ | $E_2$ bond of substituted benzene ring |
| | 259 | $1.2\times10^3$ | B bond of substituted benzene ring |

(3)

Table 3-1 IR spectral data and peak assignment for prasugrel bisulfate

| Peaks/cm$^{-1}$ | Vibration Types | Groups |
|---|---|---|
| 3010 | $\nu_{C-H}$ | Benzene ring |
| 2933, 2856 | $\nu_{C-H}$ | $CH_3$, $CH_2$ |
| 1759 | $\nu_{C=O}$ | Ester group |
| 1712 | $\nu_{C=O}$ | Ketone group |
| 1614, 1587, 1495 | $\nu_{C=C}$ | Benzene ring |
| 1453, 1373 | $\delta_{C-H}$ | $CH_3$, $CH_2$ |
| 1195 | $\nu_{C=O}$ | Ester group |
| 768 | $\delta_{C-H}$ | Benzene ring |

(4)

Table 4-1 $^1$H-NMR data of prasugrel bisulfate in DMSO-$d_6$+$D_2O$

| No. | Chemical Shifts δ | Multiplicity | No. of Protons | gCOSY |
|---|---|---|---|---|
| 1' | 11.088 | s | 1 | |
| $NH^+$ | 10.924 | s | 1 | |
| 19 | 7.686 | m | 1 | |
| 21 | 7.563 | m | 1 | |
| 22 | 7.495 | m | 1 | |
| 20 | 7.437 | m | 1 | |
| 7 | 6.585 | s | 1 | |
| 13 | 6.102 | s | 1 | |
| 1 | 4.075 | s | 2 | |
| 3 | 3.495 | s | 2 | 3.054 |
| 4 | 3.054 | s | 2 | 3.495 |
| 12 | 2.288 | s | 3 | |
| 15 | 1.914 | m | 1 | 1.151, 1.088, 0.938 |
| 17 | 1.151, 1.088 | m | 2 | 0.938, 1.914 |
| 16 | 0.938 | m | 2 | 1.151, 1.088, 1.914 |

(5)

Table 4-2 $^{13}$C-NMR data of prasugrel bisulfate in DMSO-$d_6$+$D_2O$ (ppm)

| No. | Chemical Shifts δ | Multiplicity | gHMQC | gHMBC |
|---|---|---|---|---|
| 14 | 201.994 | C | | 1.914 |
| 11 | 167.729 | C | | 2.288 |
| 23 | 160.856 | C | | 7.686, 7.563, 7.495, 7.437 |
| 6 | 149.783 | C | | 2.288 |
| 19 | 133.720 | CH | 7.686 | 7.563, 7.437 |
| 21 | 131.950 | CH | 7.563 | 7.437, 7.686 |
| 20 | 125.874 | CH | 7.437 | 7.495, 7.563, 7.686 |
| 8 | 124.188 | C | | 6.585 |
| 9 | 123.608 | C | | 3.054 |
| 22 | 116.806 | CH | 7.495 | 7.686, 7.437 |
| 18 | 115.099 | C | | 7.686, 7.495 |
| 7 | 111.902 | CH | 6.585 | |
| 13 | 69.013 | CH | 6.102 | 7.495 |
| 1 | 64.844 | $CH_2$ | 4.075 | |

(continued)

| No. | Chemical Shifts δ | Multiplicity | gHMQC | gHMBC |
|---|---|---|---|---|
| 3 | 64.844 | $CH_2$ | 3.495 | |
| 4 | 21.597 | $CH_2$ | 3.054 | |
| 12 | 20.327 | $CH_3$ | 2.288 | |
| 15 | 19.598 | CH | 1.914 | |
| 16 | 12.961 | $CH_2$ | 0.938 | 1.914, 1.151, 1.088 |
| 17 | 12.610 | $CH_2$ | 1.151, 1.088 | 1.914, 0.938 |

(6)

Table 5-1 Peak of molecular ion of prasugrel bisulfate and its assignment (MS)

| | Sample | Assignment |
|---|---|---|
| Peak of molecular ion | 374.1 | $[M-HSO_4+H]^+$ |

The present invention further provides a method for preparing prasugrel bisulfate. Prasugrel bisulfate of the present invention may be prepared by reacting 2-acetyloxy-5-(α-cycloproylcarbonyl-2-fluorobenzyl)-4,5,6,7-tetrahydrothieno[3,2-C] pyridine(prasugrel)with sulfuric acid:

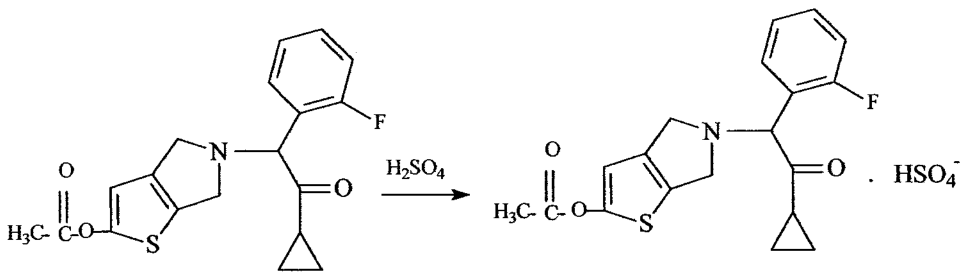


(I) (II)

A method for preparing prasugrel bisulfate of the present invention comprises the steps of:

1) Dissolving 2-acetyloxy-5-(α-cycloproylcarbonyl-2-fluorobenzyl)-4,5,6,7-tetrahydrothieno[3,2-C]pyridine in a solvent;
2) Cooling down to desired reaction temperature under stirring, and adding dropwise concentrated sulfuric acid or a mixture of concentrated sulfuric acid and a solvent under stirring; and
3) Maintaining the temperature while stirring until the reaction is completed.

In one embodiment of the present invention, the reaction temperature in step 2) is from -50°C to 30°C, and the duration of the reaction is from 10 minutes to 24 hours.
Preferably, the reaction temperature in step 2) is from -35°C to 0°C, and the duration of the reaction is from 10 minutes to 8 hours.
More preferably, the reaction temperature in step 2) is from -30°C to -15°C, and the duration of the reaction is from 10 minutes to 5 hours.
In one embodiment of the present invention, the molar ratio of prasugrel and concentrated sulfuric acid is 1 : 1-1.8.
In one embodiment of the present invention, the concentrated sulfuric acid or the mixture of concentrated sulfuric acid and the solvent in step 2) may added dropwise in one or more portions.
In the method of the present invention for preparing prasugrel bisulfate, the solvent in step 1) may be one or more selected from, but not limited to, aromatic hydrocarbons, aliphatic hydrocarbons, halogenated hydrocarbons, esters, ethers, ketones, alcohols and nitriles. Any solvent which may dissolve the reactants while not impeding the reaction for preparing prasugrel bisulfate according to the present invention may be employed.
In one embodiment of the present invention, the solvent in step 1) may be one or more selected from benzene,

toluene, xylene, ethane, cyclohexane, dichloromethane, chloroform, ethyl acetate, ethyl ether, tetrahydrofuran, petroleum ether, acetone, butanone, methanol, ethanol, acetonitrile and DMF.

Preferably, the solvent in step 1) may be one or more selected from tetrahydrofuran, acetone, methanol, ethyl ether and butanone.

More preferably, the solvent in step 1) may be one or more selected from acetone, ethyl ether and methanol.

After the reaction is completed, the target product may be obtained with a conventional process. In one embodiment of the present invention, crystals are precipitated after the reaction is completed and the product is obtained with filtration.

In another embodiment of the present invention, the solvents are evaporated in vacuum after the reaction is completed and the target product is obtained after cooling and crystallization.

In order to further purify the product, a purification process such as recrystallization or column chromatography may be employed.

In one embodiment, the product obtained is a mixture of various crystal forms. Where the product in a single crystal form is desired, a seed crystal of the desired single crystal form may be added, and the mixture is allowed to stand for crystallizaition.

The present invention further provides a pharmaceutical composition comprising prasugrel bisulfate or prasugrel bisulfate and a pharmaceutical auxiliary material, wherein the pharmaceutical auxiliary material is selected from, but not limited to, excipient, disintegrating agent, adhesive agent, lubricant, antioxidant, coating agent, colorant, flavoring agent and surfactant. Any pharmaceutical auxiliary material may be employed as long as it does not affect the activities of prasugrel bisulfate.

In one embodiment of the present invention, the pharmaceutical composition of the present invention may be prepared with a conventional process.

In one embodiment of the present invention, the pharmaceutical composition of the present invention may be in the form of, but not limited to, granules, capsules, tablets, injection solutions, infusion solutions, or suppository. It may be administered orally or parenterally. Its dosage may vary according to the drug. For an adult, a dosage of 1-1000 mg per day is appropriate. When administered orally, the compound is firstly mixed with a conventional pharmaceutical auxiliary material, and made into granules, capsules or tablets for administration. When administered parenterally, it may be administered in the form of injection solutions, infusion solutions, or suppository. The above formulations may be prepared with conventional processes.

The present invention also provides use of prasugrel bisulfate or a pharmaceutical composition thereof in preparing a medicament for preventing or treating a disease associated with thrombosis or embolism.

The present invention also provide a method for preventing or treating a disease associated with thrombosis or embolism with prasugrel bisulfate or apharmaceutical composition thereof, comprising administration of an effective amount of prasugrel bisulfate or a pharmaceutical composition thereof to a patient in need thereof.

The term "an effective amount" as used herein refers to the amount of prasugrel bisulfate or a pharmaceutical composition thereof which allows prasugrel bisulfate or the pharmaceutical composition thereof to produce desired effects in the patient.

Prasugrel bisulfate or a pharmaceutical composition thereof has good stability, oral absorbability, bioavailability, metabolic activity and platelet aggregation inhibition effect, and low toxicity, and has an effect of preventing or treating diseases associated with thrombosis or embolism. It is preferably used for preventing or treating thrombosis or embolism. The above drug may be widely used in warm blooded animal, preferably in human.

It has been demonstrated by experiments that, after administered by gastric gavage, rats administered with prasugrel bisulfate showed significantly lower rate of hemorrhage than those administered with prasugrel maleate, which demonstrated that the rate of hemorrhage and side effects are reduced after the administration of prasugrel bisulfate. It has been demonstrated by experiments that prasugrel bisulfate has very significant inhibition to platelet aggregation induced by ADP and collagen, which showed no significant difference to prasugrel maleate, suggesting that prasugrel bisulfate achieves the effects comparable to that of the prior art drug, or even better than that of prasugrel maleate to certain extent. Additionally, it has also been demonstrated by experiments that the bioavailability of prasugrel bisulfate in beagle dogs is better than that of prasugrel hydrochloride.

## Examples

The beneficial effects of the present invention will be further described with the following examples. It shall be understood that these examples are only intended to illustrate, while not limiting the scope of the present invention. Meanwhile, any obvious changes and modifications which may be made by a person skilled in the art are also encompassed in the scope of the present invention. All the references cited in the present invention are incorporated herein in their entirety by reference.

**Example 1:** Preparation of compound (II): 2-acetyloxy-5-(α-cycloproylcarbonyl-2-fluorobenzyl)-4,5,6,7-tetrahydrothieno [3,2-C]pyridine bisulfate

8 g of refined 2-acetyloxy-5-(α-cycloproylcarbonyl-2-fluorobenzyl)-4,5,6,7-tetrahydrothieno[3,2-C]pyridine is dissolved in 50 ml of acetone, and cooled to -32~-28°C. The same temperature is maintained while adding dropwise 2.5 ml of concentrated sulfuric acid under stirring over 1.5 h. After the addition is completed, the mixture is stirred under the same temperature for further 4 h, while a large amount of crystals precipitate. The reaction is stopped when no more crystals are precipitated. The mixture is filtered, and the filter cake is washed with ice-cold acetone and then dried in vacuum under 60°C to obtain 8.2 g of white crystals. Yield: 78%.

Melting point of the crystals: mp: 145-148°C

Confirmation of the structure of the crystals:

(1)

Table 1-1 Percent contents of elements C, H, N, S, F in prasugrel bisulfate sample

| Elements | Samples | | Theoretical Values |
|---|---|---|---|
| | 1 | 2 | |
| C | 50.82 | 50.83 | 51.05 |
| H | 4.53 | 4.48 | 4.50 |
| N | 3.04 | 3.08 | 2.98 |
| S | 13.87 | 13.90 | 13.63 |
| F | 3.92 | 3.89 | 4.04 |

(2)

Table 2-2 UV spectral data of prasugrel bisulfate sample

| Solvents | $\lambda_{max}$/nm | $\varepsilon_{max}$/L·mol$^{-1}$·cm$^{-1}$ | Peak Assignment |
|---|---|---|---|
| Water | 212 | $3.9\times10^3$ | $E_2$ bond of substituted benzene ring |
| | 259 | $1.5\times10^3$ | B bond of substituted benzene ring |
| 0.1 mol/L HCl | 213 | $4.0\times10^3$ | $E_2$ bond of substituted benzene ring |
| | 213 | $1.2\times10^3$ | B bond of substituted benzene ring |
| 0.1 mol/L NaOH | 214 | $4.4\times10^3$ | $E_2$ bond of substituted benzene ring |
| | 259 | $1.3\times10^3$ | B bond of substituted benzene ring |
| Methanol | 214 | $4.8\times10^3$ | $E_2$ bond of substituted benzene ring |
| | 259 | $1.2\times10^3$ | B bond of substituted benzene ring |

(3)

Table 3-1 IR spectral data and peak assignment for prasugrel bisulfate sample

| Peaks/cm$^{-1}$ | Vibration Types | Groups |
|---|---|---|
| 3010 | $\nu_{C-H}$ | Benzene ring |
| 2933,2856 | $\nu_{C-H}$ | $CH_3$, $CH_2$ |
| 1759 | $\nu_{C=O}$ | Ester group |
| 1712 | $\nu_{C-O}$ | Ketone group |
| 1614, 1587,1495 | $\nu_{C=C}$ | Benzene ring |
| 1453, 1373 | $\delta_{C-H}$ | $CH_3$, $CH_2$ |
| 1195 | $\nu_{C-O}$ | Ester group |
| 768 | $\delta_{C-H}$ | Benzene ring |

(4)

Table 4-1 $^1$H-NMR data of prasugrel bisulfate sample in DMSO-$d_6$+$D_2O$

| No. | Chemical Shifts δ | Multiplicity | No. of Protons | gCOSY |
|---|---|---|---|---|
| 1' | 11.088 | s | 1 | |
| $NH^+$ | 10.924 | s | 1 | |
| 19 | 7.686 | m | 1 | |
| 21 | 7.563 | m | 1 | |
| 22 | 7.495 | m | 1 | |
| 20 | 7.437 | m | 1 | |
| 7 | 6.585 | s | 1 | |
| 13 | 6.102 | s | 1 | |
| 1 | 4.075 | s | 2 | |
| 3 | 3.495 | s | 2 | 3.054 |
| 4 | 3.054 | s | 2 | 3.495 |
| 12 | 2.288 | s | 3 | |
| 15 | 1.914 | m | 1 | 1.151, 1.088, 0.938 |
| 17 | 1.151,1.088 | m | 2 | 0.938, 1.914 |
| 16 | 0.938 | m | 2 | 1.151, 1.088, 1.914 |

(5)

Table 4-2 $^{13}$C-NMR data of prasugrel bisulfate sample in DMSO-$d_6$+$D_2O$ (ppm)

| No. | Chemical Shifts δ | Multiplicity | gHMQC | gHMBC |
|---|---|---|---|---|
| 14 | 201.994 | C | | 1.914 |
| 11 | 167.729 | C | | 2.288 |
| 23 | 160.856 | C | | 7.686, 7.563, 7.495, 7.437 |
| 6 | 149.783 | C | | 2.288 |
| 19 | 133.720 | CH | 7.686 | 7.563,7.437 |
| 21 | 131.950 | CH | 7.563 | 7.437,7.686 |
| 20 | 125.874 | CH | 7.437 | 7.495, 7.563, 7.686 |
| 8 | 124.188 | C | | 6.585 |
| 9 | 123.608 | C | | 3.054 |
| 22 | 116.806 | CH | 7.495 | 7.686, 7.437 |
| 18 | 115.099 | C | | 7.686,7.495 |
| 7 | 111.902 | CH | 6.585 | |
| 13 | 69.013 | CH | 6.102 | 7.495 |
| 1 | 64.844 | $CH_2$ | 4.075 | |
| 3 | 64.844 | $CH_2$ | 3.495 | |
| 4 | 21.597 | $CH_2$ | 3.054 | |
| 12 | 20.327 | $CH_3$ | 2.288 | |
| 15 | 19.598 | CH | 1.914 | |
| 16 | 12.961 | $CH_2$ | 0.938 | 1.914, 1.151, 1.088 |
| 17 | 12.610 | $CH_2$ | 1.151, 1.088 | 1.914, 0.938 |

(6)

Table 5-1 Peak of ion of prasugrel bisulfate sample and its assignment (MS)

| | Sample | Assignment |
|---|---|---|
| Peak of molecular ion | 374.1 | $[M-HSO_4+H]^+$ |

**Example 2:** Preparation of compound (1): 2-acetyloxy-5-(α-cycloproylcarbonyl-2-fluorobenzyl)-4,5,6,7-tetrahydrothieno [3,2-C]pyridine bisulfate

8 g of 2-acetyloxy-5-(α-cycloproylcarbonyl-2-fluorobenzyl)-4,5,6,7-tetrahydrothieno[3,2-C]pyridine is dissolved in 50 ml of acetone, and cooled to -14~-16°C. The same temperature is maintained while adding dropwise a mixture of 2.5 ml of concentrated sulfuric acid and 30 ml of acetone under stirring in two portions, in which 18 ml is added dropwise over 10 min and stirred for 1.5 h under the same temperature, and the remaining sulfuric acid solution is then added dropwise over 1 h. The mixture is stirred under the same temperature for 3 h, filtered to obtain the precipitated crystals. The filter cake is washed with ice-cold acetone, and then dried in vacuum under 60°C to obtain 7.6 g of white crystals. Yield: 72.3%.

Melting point of the crystals: mp: 148-155°C

Confirmation of the structure of the crystals:

(1)

Table 1-1 Percent contents of elements C, H, N, S, F in prasugrel bisulfate sample

| Elements | Samples | | Theoretical Values |
|---|---|---|---|
| | 1 | 2 | |
| C | 50.93 | 51.02 | 51.05 |
| H | 4.50 | 4.49 | 4.50 |
| N | 3.01 | 3.02 | 2.98 |
| S | 13.68 | 13.65 | 13.63 |
| F | 4.04 | 4.02 | 4.04 |

(2)

Table 2-2 UV spectral data of prasugrel bisulfate sample

| Solvents | $\lambda_{max}$/nm | $\varepsilon_{max}$/$L \cdot mol^{-1} \cdot cm^{-1}$ | Peak Assignment |
|---|---|---|---|
| Water | 212 | $3.9 \times 10^3$ | $E_2$ bond of substituted benzene ring |
| | 259 | $1.5 \times 10^3$ | B bond of substituted benzene ring |
| 0.1 mol/L HCl | 213 | $4.0 \times 10^3$ | $E_2$ bond of substituted benzene ring |
| | 213 | $1.2 \times 10^3$ | B bond of substituted benzene ring |
| 0.1 mol/L NaOH | 214 | $4.4 \times 10^3$ | $E_2$ bond of substituted benzene ring |
| | 259 | $1.3 \times 10^3$ | B bond of substituted benzene ring |
| Methanol | 214 | $4.8 \times 10^3$ | $E_2$ bond of substituted benzene ring |
| | 259 | $1.2 \times 10^3$ | B bond of substituted benzene ring |

(3)

Table 3-1 IR spectral data and peak assignment for prasugrel bisulfate sample

| Peaks/$cm^{-1}$ | Vibration Types | Groups |
|---|---|---|
| 3010 | $\nu_{C-H}$ | Benzene ring |
| 2936, 2858 | $\nu_{C-H}$ | $CH_3$, $CH_2$ |
| 1760 | $\nu_{C=O}$ | Ester group |
| 1712 | $\nu_{C=O}$ | Ketone group |
| 1614, 1588,1495 | $\nu_{C=C}$ | Benzene ring |
| 1453, 1375 | $\delta_{C-H}$ | $CH_3$, $CH_2$ |
| 1196 | $\nu_{C-O}$ | Ester group |
| 768 | $\delta_{C-H}$ | Benzene ring |

(4)

Table 4-1 $^1$H-NMR data of prasugrel bisulfate sample in DMSO-$d_6$+$D_2O$

| No. | Chemical Shifts δ | Multiplicity | No. of Protons | gCOSY |
|---|---|---|---|---|
| 1' | 11.089 | s | 1 | |
| $NH^+$ | 10.926 | s | 1 | |
| 19 | 7.684 | m | 1 | |
| 21 | 7.565 | m | 1 | |
| 22 | 7.496 | m | 1 | |
| 20 | 7.438 | m | 1 | |
| 7 | 6.586 | s | 1 | |
| 13 | 6.104 | s | 1 | |
| 1 | 4.078 | s | 2 | |
| 3 | 3.498 | s | 2 | 3.052 |
| 4 | 3.056 | s | 2 | 3.493 |
| 12 | 2.288 | s | 3 | |
| 15 | 1.916 | m | 1 | 1.152, 1.088, 0.936 |
| 17 | 1.152, 1.088 | m | 2 | 0.938, 1.914 |
| 16 | 0.938 | m | 2 | 1.152, 1.088, 1.914 |

(5)

Table 4-2 $^{13}$C-NMR data ofprasugrel bisulfate sample in DMSO-$d_6$+$D_2O$ (ppm)

| No. | Chemical Shifts δ | Multiplicity | gHMQC | gHMBC |
|---|---|---|---|---|
| 14 | 201.996 | C | | 1.914 |
| 11 | 167.729 | C | | 2.288 |
| 23 | 160.858 | C | | 7.686, 7.563, 7.495, 7.437 |
| 6 | 149.784 | C | | 2.288 |
| 19 | 133.722 | CH | 7.686 | 7.563, 7.437 |
| 21 | 131.952 | CH | 7.563 | 7.437, 7.686 |
| 20 | 125.874 | CH | 7.437 | 7.495, 7.563, 7.686 |
| 8 | 124.188 | C | | 6.585 |
| 9 | 123.608 | C | | 3.054 |
| 22 | 116.806 | CH | 7.495 | 7.686, 7.437 |
| 18 | 115.099 | C | | 7.686, 7.495 |
| 7 | 111.902 | CH | 6.585 | |
| 13 | 69.014 | CH | 6.102 | 7.495 |
| 1 | 64.844 | $CH_2$ | 4.075 | |
| 3 | 64.844 | $CH_2$ | 3.495 | |
| 4 | 21.597 | $CH_2$ | 3.054 | |
| 12 | 20.328 | $CH_3$ | 2.288 | |
| 15 | 19.598 | CH | 1.914 | |
| 16 | 12.962 | $CH_2$ | 0.938 | 1.914, 1.151, 1.088 |
| 17 | 12.612 | $CH_2$ | 1.151, 1.088 | 1.914,0.938 |

(6)

Table 5-1 Peak of molecular ion of prasugrel bisulfate sample and its assignment (MS)

| | Sample | Assignment |
|---|---|---|
| Peak of molecular ion | 374.1 | $[M\text{-}HSO_4+H]^+$ |

**Example 3:** Preparation of compound (II): 2-acetyloxy-5-(α-cycloproylcarbonyl-2-fluorobenzyl)-4,5,6,7-tetrahydrothieno [3,2-C]pyridine bisulfate

8 g of refined 2-acetyloxy-5-(α-cycloproylcarbonyl-2-fluorobenzyl)-4,5,6,7-tetrahydrothieno[3,2-C]pyridine is dissolved in 100 ml of ethyl ether, and cooled to -48~-30°C. The same temperature is maintained while adding dropwise 2.6 ml of concentrated sulfuric acid over 2 h. After the addition is completed, the mixture is stirred under the same temperature for further 1.5 h, while a large amount of crystals precipitate. The reaction is stopped when no more crystals are precipitated. The mixture is filtered and the filter cake is washed with ice-cold ethyl ether, and then dried in vacuum under 20°C to obtain 7.4 g of white crystals. Yield: 70.4%.

**Example 4:** Preparation of compound (II): 2-acetyloxy-5-(α-cycloproylcarbonyl-2-fluorobenzyl)-4,5,6,7-tetrahydrothieno [3,2-C]pyridine bisulfate

8 g of refined 2-acetyloxy-5-(α-cycloproylcarbonyl-2-fluorobenzyl)-4,5,6,7-tetrahydrothieno[3,2-C]pyridine is dissolved in 100 ml of ethyl ether, and cooled to -35~-5°C. The same temperature is maintained while adding dropwise 2.6 ml of concentrated sulfuric acid over 1.5 h. After the addition is completed, the mixture is stirred under the same temperature for further 2 h, while a large amount of crystals precipitate. The reaction is stopped when no more crystals are precipitated. The mixture is filtered and the filter cake is washed with ice-cold ethyl ether, and then dried in vacuum under 20°C to obtain 6.9 g of white crystals. Yield: 65.6%.

**Example 5:** Preparation of compound (II): 2-acetyloxy-5-(α-cycloproylcarbonyl-2-fluorobenzyl)-4,5,6,7-tetrahydrothieno [3,2-C]pyridine bisulfate

8 g of refined 2-acetyloxy-5-(α-cycloproylcarbonyl-2-fluorobenzyl)-4,5,6,7-tetrahydrothieno[3,2-C]pyridine is dissolved in 100 ml of ethyl ether, and cooled to -15~0°C. The same temperature is maintained while adding dropwise 2.6 ml of concentrated sulfuric acid over 1 h. After the addition is completed, the mixture is stirred under the same temperature for further 0.5 h, while crystals precipitate. The reaction is stopped when no more crystals are precipitated. The mixture is filtered and the filter cake is washed with ice-cold ethyl ether, and then dried in vacuum under 20 °C to obtain 5.6 g of white crystals. Yield: 53.3%.

**Example 6:** Preparation of compound (II): 2-acetyloxy-5-(α-cycloproylcarbonyl-2-fluorobenzyl)-4,5,6,7-tetrahydrothieno [3,2-C]pyridine bisulfate

8 g of refined 2-acetyloxy-5-(α-cycloproylcarbonyl-2-fluorobenzyl)-4,5,6,7-tetrahydrothieno[3,2-C]pyridine is dissolved in 50 ml of acetone, and cooled to -45~-40°C. The same temperature is maintained while adding dropwise a mixture of 2.6 ml of concentrated sulfuric acid and 30 ml of acetone over 1 h. After the addition is completed, the mixture is stirred under the same temperature for further 2 h, while a large amount of crystals precipitate. The reaction is stopped when no more crystals are precipitated. The mixture is filtered and the filter cake is washed with ice-cold acetone, and then dried in vacuum under 60°C to obtain 8.1 g of white crystals. Yield: 76.1%.

**Example 7:** Preparation of compound (II): 2-acetyloxy-5-(α-cycloproylcarbonyl-2-fluorobenzyl)-4,5,6,7-tetrahydrothieno [3,2-C]pyridine bisulfat

8 g of refined 2-acetyloxy-5-(α-cycloproylcarbonyl-2-fluorobenzyl)-4,5,6,7-tetrahydrothieno[3,2-C]pyridine is dissolved in 50 ml of acetone, and cooled to -35~-10°C. The same temperature is maintained while adding dropwise a mixture of 2.6 ml of concentrated sulfuric acid and 30 ml of acetone over 1.5 h. After the addition is completed, the mixture is stirred under the same temperature for further 2 h, while a large amount of crystals precipitate. The reaction is stopped when no more crystals are precipitated. The mixture is filtered and the filter cake is washed with ice-cold acetone, and then dried in vacuum under 60°C to obtain 7.7 g of white crystals. Yield: 73.3%.

**Example 8:** Preparation of compound (II): 2-acetyloxy-5-(α-cycloproylcarbonyl-2-fluorobenzyl)-4,5,6,7-tetrahydrothieno [3,2-C]pyridine bisulfate

8 g of refined 2-acetyloxy-5-(α-cycloproylcarbonyl-2-fluorobenzyl)-4,5,6,7-tetrahydrothieno[3,2-C]pyridine is dissolved in 50 ml of acetone, and cooled to 0~15 °C. The same temperature is maintained while adding dropwise a mixture of 2.6 ml of concentrated sulfuric acid and 30 ml of acetone over 1 h. After the addition is completed, the mixture is stirred under the same temperature for further 2 h, while a large amount of crystals precipitate. The reaction is stopped when no more crystals are precipitated. The mixture is filtered and the filter cake is washed with ice-cold acetone, and then dried in vacuum under 60°C to obtain 6.8 g of white crystals. Yield: 64.9%.

**Example 9:** Determination of bioavailability of prasugrel bisulfate

Six healthy male beagle dogs of around 7-month-old, without statistical difference in their weights, are used in the experiments and fed normally for 2 weeks without receiving any drug. Six beagle dogs are divided randomly into prasugrel bisulfate group and prasugrel hydrochloride group, with 3 dogs in each group. Following the process disclosed in Experimental Example 1 of Chinese Patent Publication No. CN1214031A, the pharmacokinetic parameters of S-methylate in the plasma of the dogs in each group after administration are determined (the administration dosage for both groups is 10mg/kg).

It is demonstrated by the results of the experiment that, comparing with prasugrel hydrochloride group, prasugrel bisulfate group has a significant difference, indicating that prasugrel bisulfate has a better bioavailability than prasugrel hydrochloride in beagle dogs.

Table 1 Pharmacokinetic parameters of S-methylate in the plasma of beagle dogs after administration

| Groups | n | AUC (μg•min/ml) | Cmax (μg/ml) |
|---|---|---|---|
| Prasugrel Hydrochloride | 3 | 63.7±11.0 | 0.98±0.09 |
| Prasugrel Bisulfate | 4 | 82.3±7.6* | 1.21±0.13* |

*P<0.05 comparing with prasugrel hydrochloride group

**Example 10:** Inhibition effect on platelet aggregation in rats by prasugrel bisulfate

1. Grouping and administration

Thirty male SD rats weighted 200-300g are provided by the Experimental Animal Center of Lunan Pharmaceutical Group corporation and are divided randomly into 5 groups with 10 rats in each group. The animals are acclimatized for one week before experiment. The animals in the excipient group (equivalent volume of physiological saline), prasugrel maleate group (10mg/kg), prasugrel bisulfate group (10mg/kg) are administered by gastric gavage once each day for 7 days.

2. Determination of the parameters

2.1 Determination of the inhibition effect on platelet aggregation

Two days after administered as above, the animals are starved over night. One hour after administered on the second day, the animals are anaesthetized by intraperitoneal injection of urethane, and then celiotomized to separate the abdominal aorta. A polyethylene cannula is inserted to collect 5 ml of blood into a test tube (in the tube is placed beforehand 0.5 ml of 3.8% sodium citrate solution, i.e. the ratio of the anticoagulant solution and blood is 1:9). The whole blood is centrifuged at 1000 rpm for 4 min. 1 ml of platelet-rich plasma (PRP) is taken. The remaining liquid is centrifuged at 3000 rpm for further 8 min. 1 ml of platelet-poor plasma (PPP) is taken. The two samples are placed separately in two plastic test tubes under a constant temperature of (37±0.1)°C. Using ADP (30 ul/tube) and collagen (30 ul/tube) as aggregation inducers, the maximum aggregation intensity is determined and the inhibition rate is calculated.

Rate of inhibiting aggregation = (aggregation intensity for excipient group - aggregation intensity for experimental group) / aggregation intensity for excipient group × 100%

2.2 Determination of the rate of hemorrhage

After the rate of inhibiting aggregation is determined, the rats are sacrificed and dissected. The appearances of hyperemiam, oedema, hemorrhage in the gastric mucosa are examined with the aid of a magnifier, and the rats developed with hyperemiam, oedema, hemorrhage in the gastric mucosa are recorded into corresponding test groups as cases of hemorrhage.

3. Results

It can be seen from Table 2 that prasugrel bisulfate shows very significant inhibition to platelet aggregation induced by ADP and collagen in rats, which has no significant difference comparing with prasugrel maleate, indicating that the pharmacological effect of prasugrel bisulfate achieves the level of the prior art, and is even better than that of prasugrel maleate.

It can be seen from Table 3 that, after administration by gastric gavage, the rate of hemorrhage in the prasugrel bisulfate group is significantly lower than that in the prasugrel maleate group, indicating that oral administration of prasugrel bisulfate results in low rate of hemorrhage and reduced side effects.

Table 2 Inhibition of prasugrel sulfate on platelet aggregation induced by ADP and collagen in rats

| Groups | n | ADP | | Collagen | |
|---|---|---|---|---|---|
| | | Aggregation Intensity (%) | Inhibition Rate (%) | Aggregation Intensity (%) | Inhibition Rate (%) |
| Excipient | 10 | 0.637±0.103 | | 0.753±0.117 | |
| Prasugrel Maleate | 10 | 0.352±0.089** | 45.2±12.8 | 0.394±0.070** | 47.9±12.0 |
| Prasugrel Bisulfate | 10 | 0.336±0.075** | 48.5±10.6 | 0.346±0.083** | 54.3±13.5 |

** p<0.01, comparing with control group

Table 3 Influence of prasugrel bisulfate on rate of hemorrhage in rats

| Groups | n | Cases of Hemorrhage | Rate of Hemorrhage (%) |
|---|---|---|---|
| Excipient | 10 | 0 | 0 |
| Prasugrel Maleate | 10 | 5 | 50 |
| Prasugrel Bisulfate | 10 | 1 | 10 |

**Example 11:** Preparation of prasugrel bisulfate tablets

| | |
|---|---|
| Prasugrel bisulfate | 10 g |
| Pregelatinized starch 1500 | 75 g |
| Microcrystalline cellulose 102 | 20 g |
| Magnesium stearate | 1 g |

Procedures for preparation: the above raw material is sieved with a 100 mesh sieve, and mixed homogeneously with the above raw auxiliary materials weighed according to prescribed amounts, and then pressed directly.

**Example 12:** Preparation of prasugrel bisulfate tablets

| | |
|---|---|
| Prasugrel bisulfate | 10 g |
| Sodium carboxymethyl starch | 10 g |
| Lactose 80 | 80 g |
| Magnesium stearate | 1 g |

Procedures for preparation: the above raw material is sieved with a 100 mesh sieve, and mixed homogeneously with the above auxiliary materials weighed according to prescribed amounts, and then pressed directly.

**Example 13:** Preparation of prasugrel bisulfate capsules

| | |
|---|---|
| Prasugrel bisulfate | 25 g |
| Microcrystalline cellulose | 120 g |
| β-Cyclodextrin | 20 g |
| Differential silica gel | 2 g |

Procedures for preparation: prasugrel bisulfate and β-cyclodextrin are placed into a mortar, ground and mixed homogeneously. Microcrystalline cellulose and differential silica gel are added in sequence and mixed homogeneously. The resulted mixture is filled into capsule shells.

## Claims

1. Prasugrel bisulfate, represented by the following formula (II):

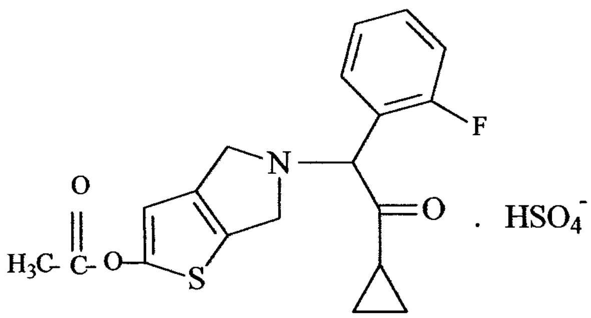

(II)

Wherein prasugrel bisulfate is a single optical isomer or a mixture of optical isomers in any proportion, or is a hydrate of prasugrel bisulfate.

2. A method for preparing prasugrel bisulfate, comprising the steps of:

1) Dissolving prasugrel in a solvent;
2) Cooling down to desired reaction temperature under stirring, and adding dropwise concentrated sulfuric acid or a mixture of concentrated sulfuric acid and a solvent under stirring; and
3) Maintaining the temperature while stirring until the reaction is completed.

3. The method according to claim 2, wherein the reaction temperature in step 2) is from -50°C to 30°C, and the duration of the reaction is from 10 minutes to 24 hours.

4. The method according to claim 3, wherein the reaction temperature in step 2) is from -35 °C to 0°C, and the duration of the reaction is from 10 minutes to 8 hours.

5. The method according to claim 4, wherein the reaction temperature in step 2) is from -30°C to -15 °C, and the duration of the reaction is from 10 minutes to 5 hours.

6. The method according to claim 2, wherein the molar ratio of prasugrel and concentrated sulfuric acid is 1 : 1-1.8.

7. The method according to claim 2, wherein the solvent in step 1) is one or more selected from benzene, toluene, xylene, ethane, cyclohexane, dichloromethane, chloroform, ethyl acetate, ethyl ether, tetrahydrofuran, petroleum ether, acetone, butanone, methanol, ethanol, acetonitrile and DMF.

8. The method according to claim 7, wherein the solvent in step 1) is one or more selected from tetrahydrofuran, acetone, methanol, ethyl ether and butanone.

9. The method according to claim 8, wherein the solvent in step 1) is one or more selected from acetone, ethyl ether and methanol.

10. The method according to claim 2, wherein the concentrated sulfuric acid or the mixture of concentrated sulfuric acid and the solvent in step 2) is added dropwise in one or more portions.

11. A pharmaceutical composition, comprising prasugrel bisulfate according to claim 1 or prasugrel bisulfate according to claim 1 and a pharmaceutical auxiliary material.

12. The pharmaceutical composition according to claim 11, wherein the pharmaceutical composition is in the form of granules, capsules, tablets, injection solutions, or suppository.

13. A use of prasugrel bisulfate according to claim 1 in preparing a medicament for preventing or treating a disease associated with thrombosis or embolism.

14. A method for preventing or treating a disease associated with thrombosis or embolism with prasugrel bisulfate according to claim 1, comprising administration of an effective amount of prasugrel bisulfate to a patient in need thereof.

## INTERNATIONAL SEARCH REPORT

International application No.
PCT/CN2009/000860

**A. CLASSIFICATION OF SUBJECT MATTER**

**See extra sheet**

According to International Patent Classification (IPC) or to both national classification and IPC

B. FIELDS SEARCHED

Minimum documentation searched (classification system followed by classification symbols)

IPC：C07D495, A61K31, A61P7

Documentation searched other than minimum documentation to the extent that such documents are included in the fields searched

Electronic data base consulted during the international search (name of data base and, where practicable, search terms used)

WPI, EPODOC, PAJ, CNPAT, CNKI, CAPLUS, REG
prasugrel, clopidogrel, pyridine, thiophene, thiofuran, cyclopropyl, benzyl, bisulphate, bisulfate, hydrosulfate, hydrogen sulfate, hydrosulphate, [7664-93-9], [150322-43-3], [120202-66-6]

C. DOCUMENTS CONSIDERED TO BE RELEVANT

| Category* | Citation of document, with indication, where appropriate, of the relevant passages | Relevant to claim No. |
|---|---|---|
| X | CN 101177430 A (LUNAN PHARMACY GROUP CO LTD), 14 May 2008 (14.05.2008), see claims 1 and 18, specification pages 1-2 | 1-13 |
| X | WO 2008060934 A (ACUSPHERE INC), 22 May 2008 (22.05.2008), see the example | 1-13 |

☐ Further documents are listed in the continuation of Box C. ☒ See patent family annex.

* Special categories of cited documents:

"A" document defining the general state of the art which is not considered to be of particular relevance

"E" earlier application or patent but published on or after the international filing date

"L" document which may throw doubts on priority claim (S) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&"document member of the same patent family

| Date of the actual completion of the international search | Date of mailing of the international search report |
|---|---|
| 20 Oct. 2009 (20.10.2009) | **05 Nov. 2009 (05.11.2009)** |

| Name and mailing address of the ISA/CN | Authorized officer |
|---|---|
| The State Intellectual Property Office, the P.R.China<br>6 Xitucheng Rd., Jimen Bridge, Haidian District, Beijing, China 100088<br>Facsimile No. 86-10-62019451 | Chang Xiaoyu<br>Telephone No. (86-10)62084403 |

Form PCT/ISA /210 (second sheet) (July 2009)

## INTERNATIONAL SEARCH REPORT

International application No.
PCT/CN2009/000860

**Box No. II Observations where certain claims were found unsearchable (Continuation of item 2 of first sheet)**

This international search report has not been established in respect of certain claims under Article 17(2)(a) for the following reasons:

1. ☒ Claims Nos.: 14
   because they relate to subject matter not required to be searched by this Authority, namely:

   Claim 14 relates to the treatment of diseases for mammal, which do not comply with the requirement of rule39.1. Because claim 13 relates to the corresponding pharmaceutical uses, the search has not been carried out with the present claim 14.

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.
2. ☐ As all searchable claims could be searched without effort justifying an additional fees, this Authority did not invite payment of additional fee.
3. ☐ As only some of the required additional search fees were timely paid by the applicant, this international search report covers only those claims for which fees were paid, specifically claims Nos.:
4. ☐ No required additional search fees were timely paid by the applicant. Consequently, this international search report is restricted to the invention first mentioned in the claims; it is covered by claims Nos.:

**Remark on protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA /210 (continuation of first sheet (2)) (July 2009)

**INTERNATIONAL SEARCH REPORT**
Information on patent family members

International application No.
PCT/CN2009/000860

| Patent Documents referred in the Report | Publication Date | Patent Family | Publication Date |
|---|---|---|---|
| CN 101177430 A | 14.05.2008 | CN 101456864 A | 17.06.2009 |
| | | CN 101343278 A | 14.01.2009 |
| WO 2008060934 A | 22.05.2008 | none | |

Form PCT/ISA /210 (patent family annex) (July 2009)

## INTERNATIONAL SEARCH REPORT

International application No.
PCT/CN2009/000860

### A. CLASSIFICATION OF SUBJECT MATTER

According to International Patent Classification (IPC) or to both national classification and IPC：

C07D 495/04 (2006.01)i
A61K 31/4365 (2006.01)i
A61P 7/02 (2006.01)n

Form PCT/ISA /210 (extra sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 1298132 A
- EP 0542411 A
- US 2004024013 A
- CN 1214031 A